# EUROPEAN PATENT APPLICATION

(11) **EP 2 246 018 A1**
(43) Date of publication of application: **03.11.2010**
(21) Application number: 08871733.5
(22) Date of filing: 17.12.2008
(51) Int. Cl.: A61F 13/15, A61F 13/472, A61F 13/494

(54) **ABSORBENT ARTICLE**

(30) Priority: 30.01.2008 JP 2008018493
(71) Applicant: Uni-charm Corporation, Ehime 799-0111 (JP)
(72) Inventor: KUDO, Jun, Kanonji-shi Kagawa 769-1602 (JP); KINOSHITA, Hideyuki, Kanonji-shi Kagawa 769-1602 (JP); HASHINO, Akira, Kanonji-shi Kagawa 769-1602 (JP)
(74) Representative: Peter, Julian
(86) International application number: PCT/JP2008/072909
(87) International publication number: WO 2009/096108

(57) **Abstract**

The present invention aims to provide an absorbent article provided with the leak-barrier walls improved so that good fitness is assured and leak sideways of excretion such as menstrual blood can be reliably prevented.

An absorbent article 1 has a shape being relatively long in a longitudinal direction and comprises a top layer, a bottom layer, an absorbent core 4 having a shape being relatively long in the longitudinal direction and a pair of leak-barrier walls 5, 5. Each of the pair of leak-barrier walls 5, 5 comprises a proximal wall section 50 adapted to stand up on the top layer, a first overhang section 51 extending outward in a transverse direction from the vicinity of the upper end of the proximal wall section 50 and provided with a plurality of first elastic members 51b attached under tension thereto so as to extend in the longitudinal direction of the absorbent article 1 and a second overhang section 52 formed below the first overhang section 51 so as to extend outward in the transverse direction from the vicinity of the upper end of the proximal wall section 50 and provided with second elastic members 52b attached under tension thereto so as to extend in the longitudinal direction of the absorbent article 1 wherein the second overhang section 52 has stiffness higher than stiffness of the first overhang section 51.

## Description

### TECHNICAL FIELD

The present invention relates to absorbent articles provided with leak barriers, for example, sanitary napkins, disposable diapers or incontinent pads.

### RELATED ART

Conventionally, absorbent articles each provided outside opposite side edges of an absorbent structure with a pair of substantially T-shaped leaf-barrier walls each comprising a proximal wall section and a pair of overhang sections extending in- and outward, respectively, from the top of the associated proximal wall section is known (See PATENT DOCUMENT 1). The absorbent article disclosed in PATENT DOCUMENT 1 intends to keep the T-shaped leak-barrier walls in close contact with the wearer's skin and thereby to prevent excretion from leaking.
However, the absorbent article disclosed in PATENT DOCUMENT 1 has a problem left unsolved behind such that the overhand sections extending inward from the tops of the respective proximal wall sections possibly cover the working surface of the absorbent article and interferes with desired absorption of excretion such as menstrual blood.
PATENT DOCUMENT 2 discloses an absorbent article provided outside opposite side edges of an absorbent structure with a pair of leak-barrier walls each comprising a proximal wall section and a plurality of overhang sections extending outward from the top of the associated proximal wall section. In the case of the absorbent article disclosed in PATENT DOCUMENT 2, the possibility that the working surface of the absorbent structure might be covered with these overhang sections can be eliminated by arranging all of the overhang sections so as to extend outward. However, it has been difficult for the disclosed absorbent article to assure that these two or more overhang sections are respectively kept in close contact with the wearer's skin.
PATENT DOCUMENT 1: Japanese Patent Application Laid-Open Publication No. 1989-68503
PATENT DOCUMENT 2: Japanese Patent Application Laid-Open Publication No. 2000-140009

### DISCLOSURE OF THE INVENTION

### PROBLEM TO BE SOLVED BY THE INVENTION

In view of the problem as has been described above, it is an object of the present invention to provide an absorbent article provided with the leak-barrier walls improved so that good fitness is assured and leak sideways of excretion such as menstrual blood can be reliably prevented.

### MEASURE TO SOLVE THE PROBLEM

Based on the findings that, in an absorbent article provided with leak-barrier walls comprising first and second overhang sections respectively including elastic members attached thereto, the object set forth above can be achieved by differentiating stiffness between the first overhang sections and the second overhang sections and differentiating tensile force between the elastic members operatively associated with the first overhang sections and the elastic members operatively associated with the second overhang sections, the inventors developed and accomplished the present invention. More specifically, the present invention provides the absorbent article defined as follows.

(1) An absorbent article having a shape being relatively long in a longitudinal direction and comprising a top layer including a top-sheet, a bottom layer including a back-sheet, an absorbent layer sandwiched between these two layers and including an absorbent core having a shape being relatively long in the longitudinal direction and a pair of leak-barrier walls provided in a vicinity of opposite side edges of the absorbent core so as to extend in the longitudinal direction of the absorbent core, the absorbent article being **characterized in that** the pair of leak-barrier walls respectively comprise proximal wall sections adapted to stand up on the top layer in a thickness direction of the absorbent article; first overhang sections extending outward in a transverse direction from the vicinity of respective upper ends of the proximal wall sections and provided with first elastic members attached under tension thereto so as to extend in the longitudinal direction of the absorbent article; and second overhang sections formed below the first overhang sections in the thickness direction so as to extend outward in the transverse direction from the vicinity of the upper ends of the proximal wall sections and provided with second elastic members attached under tension thereto so as to extend in the longitudinal direction of the absorbent article; wherein the second overhang sections have stiffness higher than stiffness of said first overhang sections.

(2) The absorbent article defined by Claim (1) wherein the first overhang sections have contractile force higher than contractile force of the second overhang sections.

(3) The absorbent article defined by Claim (1) or (2) wherein each of the first overhang sections is provided in the vicinity of a front end defined by one of opposite ends in the longitudinal direction of the first overhang section with a first front adhesive region adapted to be bonded to the top layer and in the vicinity of a rear end defined by the other of opposite ends in the longitudinal direction of the first overhang section with a first rear adhesive region adapted to be bonded to the top layer; each of the second overhang sections is provided in the vicinity of a front end defined by one of opposite ends in the longitudinal direction of the second overhang section with a second front adhesive region adapted to be bonded to the top layer and in the vicinity of a rear end defined by the other of opposite ends in the longitudinal direction of the second overhang section with a second rear adhesive region adapted to be bonded to the top layer; and a distance between the second front adhesive region and the second rear adhesive region is set to be smaller than a distance between the first front adhesive region and the first rear adhesive region.

(4) The absorbent article defined by any one of Claims (1) through (3) further including a top absorbent core being relatively long in the longitudinal direction and provided on the side of the top-sheet facing the wearer's skin so as to extend in the longitudinal direction of the absorbent article.

(5) The absorbent article defined by any one of Claims (1) through (4) wherein each of the leak-barrier walls comprises a sheet member, the first elastic members and the second elastic members; the sheet member is folded back onto itself to form a two-layered structure; the first elastic members as well as the second elastic members are arranged so as to be sandwiched between two layers in the two-layered structure; and each of the second overhang sections is formed by folding back the two-layered structure having the second elastic members sandwiched between two layers.

(6) The absorbent article defined by Claim (5) wherein the second elastic members are sandwiched between the two layers of the sheet member lying on the side facing away from the wearer's skin in the second overhang section formed by folding back he sheet member having the two-layered structure onto itself.

(7) The absorbent article defined by any one of Claims (1) through (6) wherein the second overhang section has a width larger than a width of the first overhang section.

(8) The absorbent article defined by any one of Claims (1) through (7) wherein the second overhang section has a color different from that of the first overhang section.

(9) The absorbent article defined by any one of Claims (1) through (8) wherein a second outer end of the second overhang section in its overhang direction lies at a level above a first outer end of the first overhang section in its overhang direction.

(10) The absorbent article defined by any one of Claims (1) through (9) wherein the second outer end of the second overhang section in the overhang direction is formed with a void space.

### EFFECT OF THE INVENTION

The present invention can achieve the object set forth above and provide the absorbent article improved so that good fitness is assured and leak sideways of excretion such as menstrual blood can be reliably prevented.

### BRIEF DESCRIPTION OF THE DRAWINGS

[FIG. 1] Fig. 1 is a perspective view showing a sanitary napkin as a first embodiment of an absorbent article according to the present invention as it is free from any external force.
[FIG. 2] Fig. 2 is a plan view showing a sanitary napkin free from any external force of Fig. 1 as viewed from the side of a top-sheet.
[FIG. 3] Fig. 3 is a sectional view taken along the line X-X in Fig. 2.
[FIG. 4] Fig. 4 is a scale-enlarged view showing a part of Fig. 3.
[FIG. 5] Fig. 5 is a sectional view taken along the line Y-Y in Fig. 2.
[FIG. 6] Fig. 6 is a perspective view showing a sanitary napkin as a second embodiment of the absorbent article according to the present invention as it is free from any external force.
[FIG. 7] Fig. 7 is a plan view showing the sanitary napkin free from any external force of Fig. 6 as viewed from the side of a top-sheet.
[FIG. 8] Fig. 8 is a sectional view taken along the line X-X in Fig. 7.

### IDENTIFICATION OF REFERENCE NUMERALS USED IN THE DRAWINGS

- 1: sanitary napkin
- 2: top-sheet
- 3: back-sheet
- 4: absorbent structure
- 5: leak-barrier walls
- 50: proximal wall sections
- 51: first overhang sections
- 52: second overhang sections
- 53: first front adhesive regions
- 54: first rear adhesive regions
- 55: second front adhesive regions
- 56: second rear adhesive regions
- 6: wings
- 7: leak-proof grooves
- 8: sheet members
- 9: wings' pressure-sensitive adhesive regions
- 10: main body's pressure-sensitive adhesive regions

### DETAILED DESCRIPTION OF THE PREFERRED EMBODIMENTS

Details of the absorbent article according to the present invention will be described on the basis of a first embodiment as one of preferred embodiments with reference to the accompanying drawings.
The first embodiment corresponds to the case in which the present invention is implemented in the form of sanitary napkin.

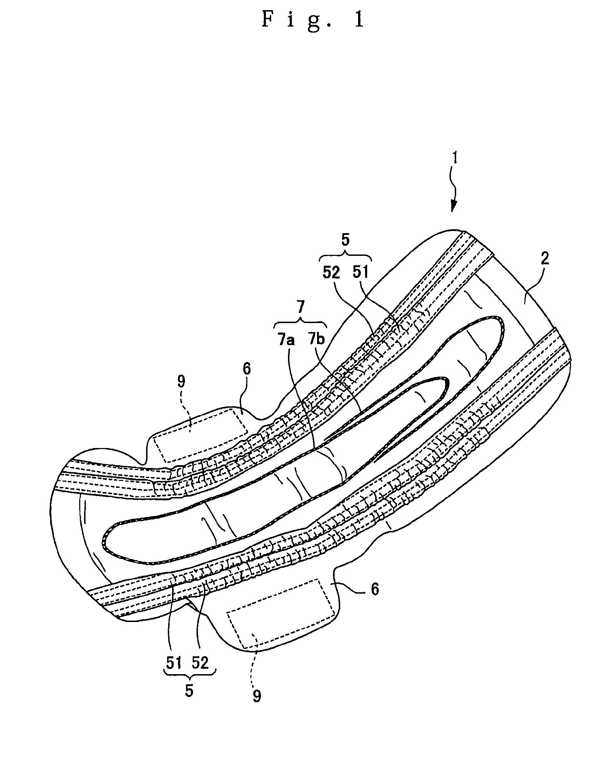

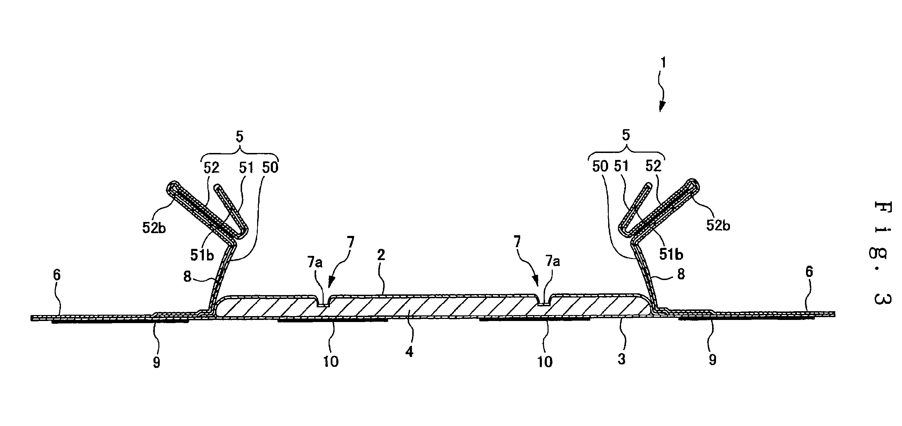

As will be apparent from Figs. 1 through 3, a sanitary napkin 1 according to the first embodiment has a shape relatively long in a longitudinal direction and comprises a liquid-pervious top-sheet 2 defining an top layer, a liquid-impervious back-sheet 3 defining a bottom layer and an absorbent core 4 sandwiched between these top- and back-sheets to define a liquid-retentive absorbent layer. Along both side edges of the sanitary napkin 1 extending in the longitudinal direction on the side of the top-sheet 2, a pair of leak-barrier walls 5, 5 is provided. As will be apparent from Fig. 3, these leak-barrier walls 5, 5 stand up on the top layer in thickness direction of the sanitary napkin 1 in the vicinity of the side edges of the absorbent core 4.

The top layer defines a side of the sanitary napkin 1 facing the wearer's skin when the sanitary napkin 1 is put on and, according to the first embodiment, the top layer primarily comprises the top-sheet 2 and a pair of sheet members 8 (See Fig. 3). The top layer further includes a second sheet (not shown) sandwiched between the top-sheet 2 and the absorbent core 4.
The bottom layer defines a side of the sanitary napkin 1 facing away from the wearer's skin when the sanitary napkin 1 is put on and, according to the first embodiment, the bottom layer primarily comprises the back-sheet 3.
The absorbent layer primarily comprises the absorbent core 4 and a core-wrapping member (not shown) adapted to wrap the absorbent core 4.

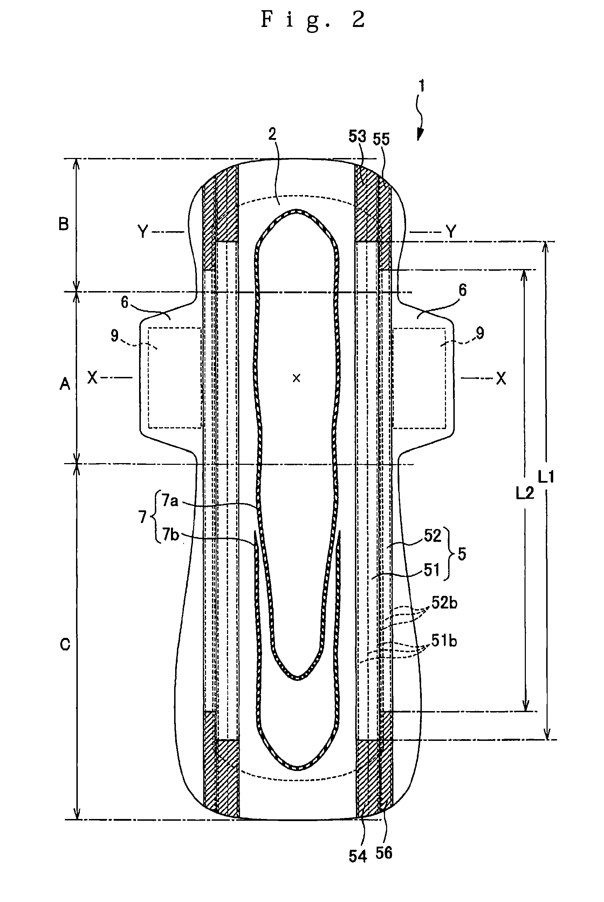

Referring to Fig. 2, the sanitary napkin 1 has a section A adapted to face the relevant excretion organ of the wearer when the sanitary napkin 1 is put on, a front section B at a distance from the section A toward the wearer's ventral side when the sanitary napkin 1 is put on and a rear section C at a distance from the section A toward the wearer' dorsal side when the sanitary napkin 1 is put on.

As shown by Figs. 2 and 3, the top-sheet 2 covers the entire upper surface of the absorbent core 4 and the back-sheet 3 covers the entire lower surface of the absorbent core 4. In the section A facing the wearer's excretion organ, the back-sheet 3 extends transversely outward beyond the side edges of the absorbent core 4 to form a pair of wings 6, 6. Also in the section A facing the wearer's excretion organ, the sheet members 8 defining the leak-barrier walls 5, 5 which will be described more in details later extend transversely outward from the side edges of the absorbent core 4 to constitute parts of the respective wings 6, 6. More specifically, the respective wings 6, 6 are formed by the respective sheet members 8 extending outward from the respective side edges of the absorbent core 4 bonded to the back-sheet 3. The top- and back-sheets 2, 3 respectively extend outward beyond front and rear ends of the absorbent core 4 and are put flat and bonded together outside the front and rear ends of the absorbent core 4.

In the respective wings 6, 6, the lower surface of the back-sheet 3 is provided with wings' pressure-sensitive adhesive regions 9, 9. In the main body section (i.e., section occupied by the absorbent core 4) of the sanitary napkin 1, the lower surface of the back-sheet 3 is provided with a pair of main body section's pressure-sensitive adhesive regions 10 extending in the longitudinal direction of the sanitary napkin 1.
The wings' pressure-sensitive adhesive regions 9 and the main body section's pressure-sensitive adhesive regions 10 are formed by coating the lower surface of the back-sheet 3 with appropriate adhesive.

Referring to Fig. 3, the absorbent core 4 and the top-sheet 2 are integrally compressed from the side of the top-sheet 2 to form leak-proof groove 7. The previously described leak-barrier walls 5, 5 lie transversely outside the leak-proof groove 7. The leak-proof groove 7 is shaped substantially symmetric about a longitudinal center line (not shown) of the sanitary napkin 1. According to the present embodiment, the leak-proof groove 7 comprises a first leak-proof groove 7a provided so as to extend from the front section B to the rear section C and a second leak-proof groove 7b in the rear section C. The first leak-proof groove 7a has an open-annular shape which is relatively long in the longitudinal direction. In the section A facing the related excretion organ, the first leak-proof groove 7a convexly curves transversely outward as will be seen in Fig. 2. The second leak-proof groove 7b is provided inside the first leak-proof groove 7a and convexly curves rearward. Front ends of the second leak-proof groove 7b are respectively positioned in the vicinity of the first leak-proof groove 7a but not contiguous to the first leak-proof groove 7a.

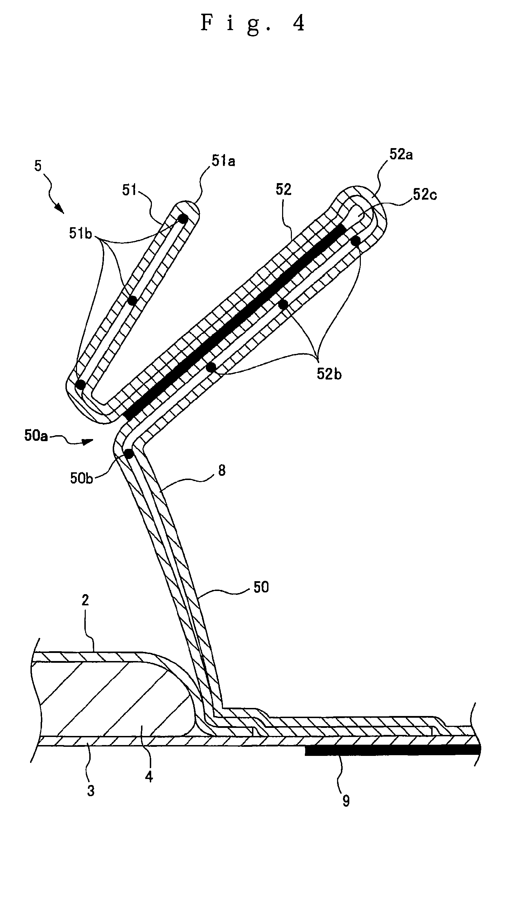

Referring to Figs. 3 and 4, each of the leak-barrier walls 5, 5 extending along the respective side edges of the sanitary napkin 1 comprises a proximal wall section 50 rising from the top layer, a first overhang section 51 extending outward in the transverse direction of the sanitary napkin 1 from the vicinity of an upper end 50a of the proximal wall section 50 and a second overhang section 52 extending outward in the width direction of the sanitary napkin 1 from the vicinity of an upper end 50a of the proximal wall section 50 so as to be formed at a level lower than the first overhang section 51.

According to the first embodiment, the second overhang section 52 has width larger than that of the first overhang section 51 as will be apparent from Figs. 3 and 4. A distal end of the second overhang section 52 which will be hereinafter referred to as a second outer end 52a lies at a level above a distal end of the first overhang section 51 which will be hereinafter referred to as a first outer end 51a.

Referring to Figs. 3 and 4, each of the leak-barrier walls 5, 5 comprises a sheet member 8 and a plurality of first elastic members 51b and a plurality of second elastic members 52b. The sheet member 8 is folded back onto itself to form a two-layered structure so that the first and second elastic members 51b, 52b may be sandwiched between two layers of the sheet member 8.
Specifically, each of the leak-barrier walls 5, 5 is formed by folding the single belt-like sheet member 8 back onto itself in the width direction so that a plurality of the first elastic members 51b and a plurality of the second elastic members 52b may be sandwiched between two layers of the sheet member 8 and then further successively folding this two-layered structure to form the first overhang section 51, the second overhang section 52 and the proximal wall section 50.

More specifically, the first overhang section 51 has its upper end corresponding to the end along which the sheet member 8 has been folded back onto itself. Between the two layers constituting the first overhang section 51, a plurality of the first elastic members 51b are attached under tension so as to extend in the longitudinal direction and to be spaced one from another by a given distance in the width direction. According to the first embodiment, the number of the first elastic members 51b is three (3).

The second overhang section 52 is formed contiguously to the first overhang section 51 which is formed, in turn, by the sheet member 8 folded back onto itself to form the two-layered structure. Also in the case of the second overhang section 52, a plurality of the second elastic members 52b are attached under tension so as to extend in the longitudinal direction and to be spaced one from another by a given distance in the width direction. It should be noted that the second overhang section 52 is formed by further folding back the two-layered structure of the sheet member 8 having the second elastic members 52b attached between two layers onto the two-layered structure of the same sheet member 8 but having none of elastic members to be put flat and bonded together. In this manner, the second overhang section 52 is formed by a four-layered structure of the sheet member 8.
The second overhang section 52 is formed by four-layered structure of the sheet member 8 in this manner and, in a consequence, the second overhang section 51 has stiffness higher than that of the first overhang section 51 formed by the two-layered structure of the sheet member 8.

According to the first embodiment, three (3) second elastic members 52b are sandwiched between the lower two layers of the total four (4) layers of the sheet member 8. In other words, a plurality of the second elastic members 52b are sandwiched between two layer of the sheet member 8 defining the two-layered structure of the four (4) layered second overhang section 52 lying on the side facing the wearer's skin. The outer end 52a of the second overhang section 52 is formed with a void space 52c as seen in Fig. 4.

The proximal wall section 50 is formed on the side opposite to the end along which the sheet member 8 has been folded back to form the two-layered sheet member 8 in continuity with the second overhang section 52. The proximal wall section 50 is provided in the vicinity of a boundary between the proximal wall section 50 and the second overhang section 52, i.e., in the vicinity of the upper end 50a of the proximal wall section 50 with a single elastic member 50b operatively associated with the proximal wall section 50.
A proximal end of the proximal wall section 50 is defined, as illustrated by Fig. 4, by the two-layered structure of the sheet member 8 is bonded to the back-sheet 3 by the intermediary of the top-sheet 2 in the vicinity of the associated side edge of the absorbent core 4. The end opposite to the end along which the sheet member 8 is folded back extends outward in the width direction beyond the associated side edge of the absorbent core and is bonded to the back-sheet 3 outside this associated side edge.

In the state free from any external force, the sanitary napkin 1 according to the first embodiment has a shape concavely curved toward the side facing the wearer's skin under contractile force of a plurality of the first elastic members 51b and a plurality of the second elastic members 52b. The expression "state free from any external force" used herein means the state of the sanitary napkin 1 free from any external force other than gravity force.

According to the first embodiment, the first elastic members 51b are attached to the sheet member 8 under a tension higher than that of the second elastic members 52b. Consequentially, the contractile force of the first overhang section 51 is higher than that of the second overhang section 52 and, in the state free from any external force other than the gravity force, the first overhang section 51 contracts more significantly than the second overhang section 52.
Significantly, the first elastic members 51b are attached to the sheet member 8 preferably under tension in a range of 110 to 240%, more preferably in a range of 120 to 200%. The second elastic members 52b are attached to the sheet member 8 under tension preferably in a range of 105 to 200%, more preferably in a range of 115 to 180%.
According to the first embodiment, even if the first and second elastic members 51b, 52b are attached to the sheet member 8 under same degree of tension, the contractile force of the first overhang section 51 can be assured to be higher than the contractile force of the second overhang section 51. This is for the reason that the second overhang section 52 is constructed to have stiffness higher than that of the first overhang section 51.

The first overhang section 51 has a width dimension preferably in a range of 3 to 20mm to assure good fitness to the wearer's body. The second overhang section 52 has a width dimension preferably in a range of 5 to 30mm to assure good fitness of the second overhang section 52 to the wearer's body outside the first overhang section 51 as viewed in the width direction. Height of the proximal wall section 50 (the length of the proximal wall section 50 unfolded) is preferably in a range of 5 to 30mm to assure its effect of preventing excretion from leaking sideways.

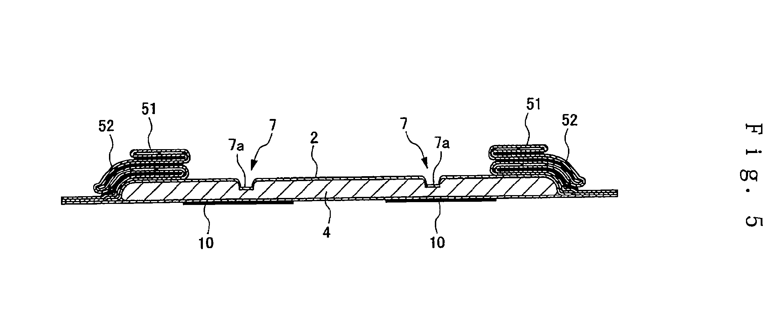

A pair of the leak-barrier walls 5, 5 is arranged, as illustrated by Figs. 2 and 5, so that respective surfaces of the first overhang section 51 and the second overhang section 52 are bonded to the top layer in the front section B and the rear section C of the sanitary napkin 1. Specifically, the first overhang section 51 has a first front adhesive region 53 adapted to be bonded to the top layer in the front section B and a first rear adhesive region 54 adapted to be bonded to top layer in the rear section C. The second overhang section 52 has a second front adhesive region 55 adapted to be bonded to the top layer in the front section B and a second rear adhesive region 56 adapted to be bonded to the top layer in the rear section C.
In the state of the sanitary napkin 1 free from any external force other than the gravity force, the first overhang section 51 stands up between the first front adhesive region 53 and the first rear adhesive region 54 while the second overhang section 52 stands up between the second front adhesive region 55 and the second rear adhesive region 56. With the leak-barrier walls standing up, a proximal end of the first overhang section 51 (i.e., a connecting region between the proximal wall section 50 and the first overhang section 51) and a proximal end of the second overhang section 52 (i.e., a connecting region between the proximal wall section 50 and the second overhang section 52) in the section A facing the relevant excretion organ lie outside the proximal end of the first overhang section 51 and the proximal end of the second overhang section 52 in the front section B and the rear section C.
According to the first embodiment, a distance L2 between the second front adhesive region 55 and the second rear adhesive region 56 is set to be smaller than a distance L1 between the first front adhesive region 53 and the first rear adhesive region 54.
In consequence, a partial length of the second overhang section 52 adapted to stand up is smaller than a partial length of the first overhang section 51 adapted to stand up.
As will be apparent from Fig. 2, the respective second front adhesive regions 55 are arranged so as to extend closer to the respective wings 6, 6 than the first front adhesive regions 53.

Now, constituent materials used for the sanitary napkin according to the first embodiment will be described.
As the top-sheet 2, for example, porous or non-porous non-woven fabric or porous plastic sheet may be used. As the back-sheet 3, for example, hydrophobic non-woven fabric, water-impervious plastic film or laminated sheet consisting of non-woven fabric and water-impervious plastic film may be used. It is also possible to use SMS-type non-woven fabric consisting of highly water-resistant melt blown non-woven fabric sandwiched between a pair of spun bonded non-woven fabric having high strength as the back-sheet.

As the absorbent core 4, for example, mixture of fluff pulp or air-laid non-woven fabric and super-absorptive polymer may be used.
As fluff pulp used for the absorbent core 4, for example, artificial cellulose fiber such as chemical pulp, cellulose fiber, rayon or acetate may be used. As an example of the air-laid non-woven fabric, thermal fusion bonded pulp and synthetic fiber subjected to thermal fusion bonding or permanently bonded by means of appropriate binder may be used. The super-absorptive polymer may be selected from a group including starch-based, acrylic acid-based or amino acid-based particulate o fibrous polymers.

As the sheet member 8, water-repellent or hydrophobic sheet member may be preferably used. Specifically, the sheet member 8 may be selected from various types of non-woven fabric such as spun laced non-woven fabric, spun bonded non-woven fabric, thermal bonded non-woven fabric, melt blown non-woven fabric, needle punched non-woven fabric and air-through non-woven fabric. Component fiber constituting the non-woven fabric may be selected from a group of synthetic fibers such as olefin-based, for example, polyester- or polypropylene-based, polyester-based or polyamide-based synthetic fibers or recycled fiber such as cupra rayon or natural fiber such as cotton.

As the first elastic members 51b, the second elastic members 52b and the elastic members 50b for the proximal wall section, thread-like or flat belt-like natural rubber or thermoplastic elastomer such as urethane, ethylene-vinyl acetate copolymer (EVA) or PE may be used so far as this is elastically stretchable and contractible thin and long material. More specifically, the useful thermoplastic elastomer may be selected from a group including polybutadiene, polyisoprene, styrene-butadiene copolymer, styrene-isopurene copolymer, polyurethane, ethylene-vinyl acetate copolymer and ethylene-αolefin copolymer each extruded into thread-like shape or slitted after film casting of such elastomer.

With the sanitary napkin 1 according to the first embodiment constructed as has been described above, the first overhang section 51 and the second overhang section 52 extending outward from the vicinity of the upper end 50a of the proximal wall section 50 in the width direction assure that excretion such as menstrual blood can be reliably prevented from leaking sideways and stiffness of the second overhang section 52 may be set to be higher than stiffness of the first overhang section 51 to improve fitness of the sanitary napkin 1 to the wearer's body.
With the sanitary napkin 1 put on, the first overhang section 51 lying above the second overhang section 52 and having its width smaller than that of the second overhang section 52 comes in close contact with the relevant excretion organ of the wearer in each of the leak-barrier walls 5, 5. The second overhang section 52 lying below the first overhang section 51 and having its width larger than that of the first overhang section 51 in each of the leak-barrier walls 5, 5 comes in close contact with the vicinity of the wearer's inguinal regions.
Stiffness of the first overhang section 51 may be set to be relatively low to facilitate the first overhang section 51 to follow delicate movement of the wearer's body in the vicinity of the relevant excretion organ. In contrast, stiffness of the second overhang section 52 may be set to be higher than stiffness of the first overhang section 51 to prevent the anxiety that the second overhang section 52 might excessively move in the vicinity of the inguinal regions apt to be subject to a relatively high load and cause excretion to leak sideways.

The second outer end 52 of the second overhang section 52 lying at a level above the first outer end 51a of the first overhang section 51 serves to improve fitness of the second overhang section 52 to the wearer's body during use of the sanitary napkin 1 and thereby to improve the preventive effect of the second overhang section 52 against leaking sideways of excretion.

The contractile force of the first overhang section 51 may be set to be higher than the contractile of the second overhang section 52 to allow the first overhang section 51 to stand up toward the wearer's body more easily than the second overhang section 52 and thereby to improve fitness of the first overhang section 51 to the wearer's body during use of the sanitary napkin 1.

The first overhang section 51 and the second overhang section 52 extend outward in the width direction and respective surfaces of these overhang sections 51, 52 remain bonded to the top layer in the front section B and the rear section C. Therefore, it is assured that the first overhang section 51 and the second overhang section 52 are kept to extend outward in the width direction and prevented from collapse inward in the width direction and covering the top surface of the absorbent layer.

The distance L2 between the second front adhesive region 55 and the second rear adhesive region 56 is set to be smaller than the distance L1 between the first front adhesive region 53 and the first rear adhesive region 54 to facilitate the second overhang section 52 to extend outward in the width direction of the sanitary napkin 1. The first front adhesive region 53 and the first rear adhesive region 54 in the first overhang section 51 are bonded to the second overhang section 52, facilitating no only the second overhang section 52 but also the first overhang section 51 to extend outward in the width direction of the sanitary napkin 1.
The second front adhesive regions 55, 55 are arranged to extend closer to the respective wings 6, 6 so that force used to fold back the wings 6, 6 may be efficiently transmitted to the respective second overhang sections 52 when the sanitary napkin 1 is put on. In consequence, the second overhang sections 52 are smoothly opened outward as the sanitary napkin 1 is put on and, once the sanitary napkin 1 has been put on, there is substantially no possibility that these second overhang sections 52 might collapse inward even if any force is exerted thereon from the outside in the width direction of the sanitary napkin 1.
With a pair of the leak-barrier walls 5 standing up on the top layer, the contractile force of the first overhang sections 51 set to be higher than the contractile force of the second overhang sections 52 functions to keep the proximal wall sections 50 standing up substantially in upright postures. In addition, the proximal ends of the respective proximal wall sections 50 lie outside the inner side edges of the first front adhesive regions 53 and the first rear adhesive regions 54 as viewed in the width direction. Such arrangement assures that the vicinity of the upper ends 50a of the respective proximal wall sections 50 are defined outside the respective inner side edges of the first front adhesive regions 53 as well as outside the respective inner side edges of the first rear adhesive regions 54 as viewed in the width direction. In this way, each of the leak-barrier walls 5 is facilitated to maintain its posture overhanging outward in the width direction and thereby to restrict a possibility that the leak-barrier wall 5 might collapse on the working surface of the absorbent core and interfere with absorption of excretion such as menstrual blood.

Each of the first overhang section 51 is formed by two-layered structure of the sheet member 8 and each of the second overhang sections 52 is formed by four-layered structure of the sheet member 8 obtained by folding back the two-layered sheet member 8 onto itself so that the second overhang section 52 may have stiffness higher than stiffness of the first overhang section 51. Such arrangement makes it possible to form the leak-barrier walls 5 according to the first embodiment from one and same sheet member 8 and thereby to improve productivity of the sanitary napkin 1.
A plurality of the second elastic members 52b are sandwiched between two layers lying on the side facing away from the wearer's skin in the second overhang section 52 formed by four-layered structure of the sheet member 8. More specifically, the second elastic members 52 are not sandwiched between two layers of the sheet member 8 lying on the side facing the wearer's skin in the second overhang section 52. Consequentially, texture experienced by the wearer when the second overhang sections 52 come in contact with the wearer's skin is thereby improved.

The second outer end 52a of the second overhang section 52 is formed with the void space 52c serving to improve fitness of the second overhang section 52 to the wearer's body.

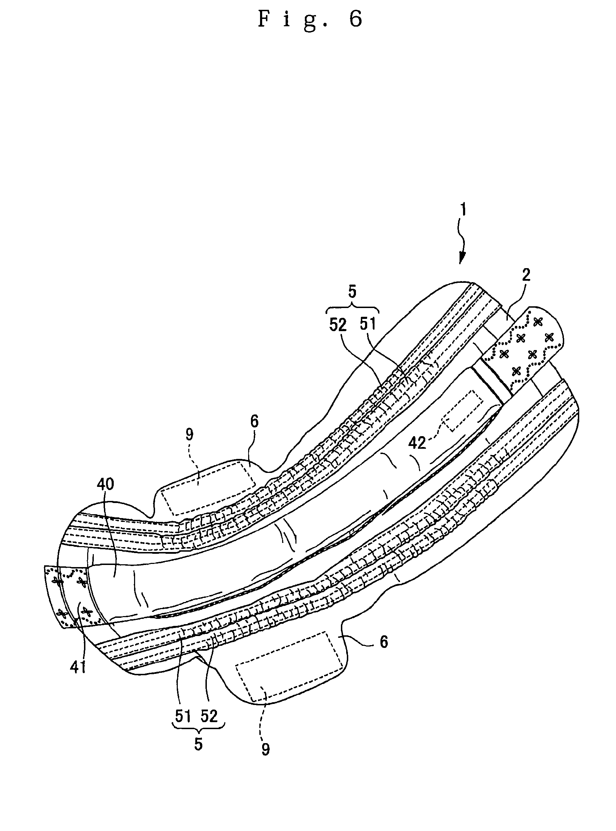

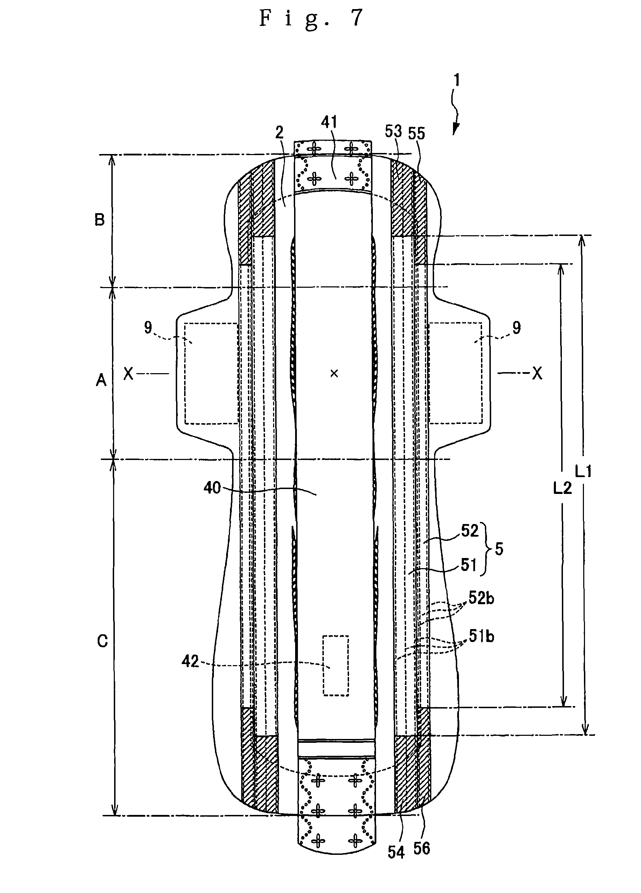

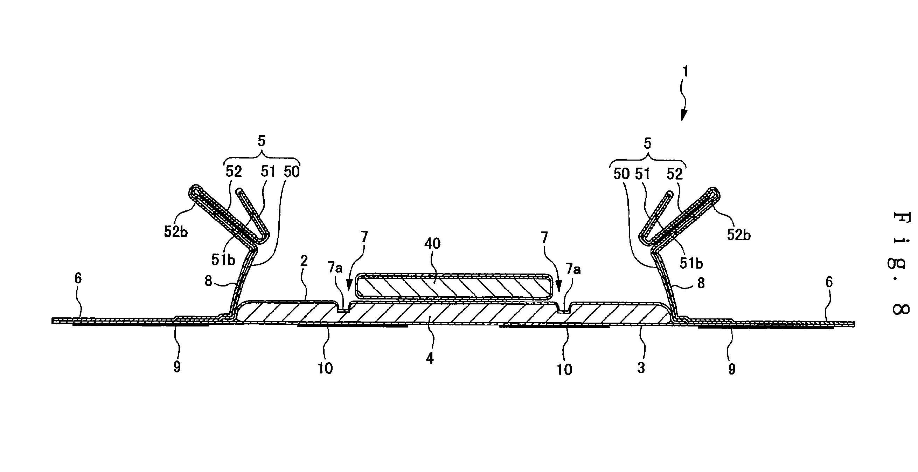

Now the absorbent article according to a second embodiment of the present invention will be described with reference to Figs. 6 through 8. Description will be limited to the aspect of this second embodiment differing from the first embodiment and the components same as or similar to those in the first embodiment will be designated with same or similar reference numerals and will not be repetitively described. The features not otherwise specified may be construed in reference to the description of the first embodiment.

The sanitary napkin 1 according to the second embodiment is similar to the sanitary napkin 1 according to the first embodiment except that the sanitary napkin 1 according to the second embodiment includes a top absorbent core 40 provided on the side of the top-sheet 2 facing the wearer's skin.
The top absorbent core 40 has a shape which is relatively long in the longitudinal direction and, as will be apparent from Figs. 6 through 8, located on the side of the top-sheet 2 facing the wearer' s skin so as to extend across a transversely middle section of the sanitary napkin 1 along the longitudinal direction of the sanitary napkin 1. The top absorbent core 40 has one end bonded to the top-sheet 2 in the front section B of the sanitary napkin 1 to form a fixed region 41. An end of the top absorbent core 40 opposite to the fixed region 41 is left as a free end. The top absorbent core 40 is provided on the side of the free end with a temporary fixing region in which the top absorbent core 40 can be temporarily fixed to the top-sheet 2. The sanitary napkin 1 according to the second embodiment allows the top absorbent core 40 to be properly put in contact with the wearer's excretion organ by temporarily attaching the temporary fixing region 41 to a desired region of the top-sheet 2.

The sanitary napkin 1 according to the second embodiment provides, in addition to the same effects as those provided by the sanitary napkin according to the first embodiment, further improves the absorption capacity of the sanitary napkin 1 for excretion such as menstrual blood by putting the top absorbent core provided on the side of the top-sheet facing the wearer's skin in closer contact with the wearer's excretion organ.

In the sanitary napkin 1 according to the second embodiment, the top absorbent core provided on the side of the top-sheet facing the wearer's skin is apt to space the top-sheet 2 from the wearer's skin. However, each of the leak-barrier walls 5, 5 comprises the proximal wall section 50 and the first and second overhang sections 1, 2 respectively extending outward from the vicinity of the upper end 50a of the proximal wall section 50 in the transverse direction. This arrangement assures that the first and second overhang sections 51, 52 are properly put in close contact with the wearer' s body to prevent excretion such as menstrual blood from leaking sideways even when the top-sheet 2 is slightly spaced from the wearer's skin.

The present invention is not limited to the embodiments as have been described hereinabove and may be modified or varied in various manners without departing from scope and spirit of the present invention.

For example, the second overhang sections 52 may be implemented to have a color different from that of the first overhang sections 51. By implementing the second overhang sections 52 to have the color different from that of the first overhang sections 51, the wearer can easily determine which of the first and second overhang sections 51, 52 have been stained with excretion such as menstrual blood when one or both of the leak-barrier walls 5, 5 has or have been stained with excretion such as menstrual blood. In this way, a level at which the sanitary napkin 1 has been stained with excretion such as menstrual blood can be easily recognized.
As one of the methods for differentiating the first and second overhang sections 51, 52 by coloring, a plurality of the second elastic members 52b operatively associated with the second overhang sections 52 may be dyed in color different from color with which a plurality of the first elastic members 51b operatively associated with the first overhang sections 51.

According to the first and second embodiments, the contractile force of the first overhang sections 51 is set to be higher than the contractile force of the second overhang sections 52 by differentiating the elongation percentage of the first elastic members 51b and the second elastic members 52b to be attached to the sheet member 8. However, the present invention is not limited to these embodiments but may be implemented in a manner that the contractile force of the first overhang sections 51 is set to be higher than the contractile force of the second overhang sections 52 by setting the number of the first elastic members 51b operatively associated with the first overhang sections 51 to be larger than the number of the second elastic members 52b operatively associated with the second overhang sections 52. Alternatively, the contractile force of the first overhang sections 51 may be set to be higher than the contractile force of the second overhang sections 52 by dimensioning a diameter of the respective first elastic members 51b operatively associated with the first overhang sections 51 to be larger than a diameter of the respective second elastic members 52b operatively associated with the second overhang sections 52.
While the second overhang sections 52 are bonded to the top layer in the second front adhesive regions 55 and the second rear adhesive regions 56 according to the first and second embodiments, the second overhang sections 52 may be additionally provided in the section A facing the wearer's excretion organ and including a pair of the wings 6, 6 with second middle adhesive regions (not shown) adapted to be respectively bonded to the top layer. In this way, a force used to fold back a pair of the wings 6, 6 to put the sanitary napkin 1 on the wearer's body may be efficiently transmitted to the second overhang sections 52. Consequently, the second overhang sections 52 are further facilitated to be opened outward when the sanitary napkin 1 is put on and, once the sanitary napkin has been put on, there is substantially no anxiety that the second overhang sections 52 might collapse even under a force exerted thereon from the outside in the transverse direction.

While the leak-barrier walls 5 as well as the wings 6 are integrally formed by the single sheet member 8 according to the first and second embodiments, it is possible to form the wings 6 by a sheet other than the sheet member 8 constituting the leak-barrier walls 5.

The absorbent article according to the present invention is not limited to the sanitary napkin but may be implemented also, for example, in the form of incontinent pad, panty liner or disposable diaper.

## Claims

1. An absorbent article having a shape being relatively long in a longitudinal direction and comprising a top layer including a top-sheet, a bottom layer including a back-sheet, an absorbent layer sandwiched between these two layers and including an absorbent core having a shape being relatively long in the longitudinal direction and a pair of leak-barrier walls provided in a vicinity of opposite side edges of said absorbent core so as to extend in the longitudinal direction of said absorbent core, said absorbent article being **characterized in that**:
said pair of leak-barrier walls respectively comprise
proximal wall sections adapted to stand up on said top layer in a thickness direction of said absorbent article;
first overhang sections extending outward in a transverse direction from the vicinity of respective upper ends of said proximal wall sections and provided with first elastic members attached under tension thereto so as to extend in the longitudinal direction of said absorbent article; and
second overhang sections formed below said first overhang sections in said thickness direction so as to extend outward in said transverse direction from the vicinity of said upper ends of said proximal wall sections and provided with second elastic members attached under tension thereto so as to extend in the longitudinal direction of said absorbent article;
wherein said second overhang sections have stiffness higher than stiffness of said first overhang sections.

2. The absorbent article defined by Claim 1 wherein said first overhang sections have contractile force higher than contractile force of said second overhang sections.

3. The absorbent article defined by Claim 1 or 2 wherein:
each of said first overhang sections is provided in the vicinity of a front end defined by one of opposite ends in said longitudinal direction of said first overhang section with a first front adhesive region adapted to be bonded to said top layer and in the vicinity of a rear end defined by the other of opposite ends in said longitudinal direction of said first overhang section with a first rear adhesive region adapted to be bonded to said top layer;
each of said second overhang sections is provided in the vicinity of a front end defined by one of opposite ends in said longitudinal direction of said second overhang section with a second front adhesive region adapted to be bonded to said top layer and in the vicinity of a rear end defined by the other of opposite ends in said longitudinal direction of said second overhang section with a second rear adhesive region adapted to be bonded to said top layer; and
a distance between said second front adhesive region and said second rear adhesive region is set to be smaller than a distance between said first front adhesive region and said first rear adhesive region.

4. The absorbent article defined by any one of Claims 1 through 3 further including a top absorbent core being relatively long in the longitudinal direction and provided on the side of said top-sheet facing the wearer's skin so as to extend in the longitudinal direction of said absorbent article.

5. The absorbent article defined by any one of Claims 1 through 4 wherein:
each of said leak-barrier walls comprises a sheet member, said first elastic members and said second elastic members;
said sheet member is folded back onto itself to form a two-layered structure;
said first elastic members as well as said second elastic members are arranged so as to be sandwiched between two layers in said two-layered structure; and
each of said second overhang sections is formed by folding back said two-layered structure having said second elastic members sandwiched between two layers.

6. The absorbent article defined by Claim 5 wherein said second elastic members are sandwiched between said two layers of said sheet member lying on the side facing away from the wearer's skin in said second overhang section formed by folding back said sheet member having said two-layered structure onto itself.

7. The absorbent article defined by any one of Claims 1 through 6 wherein said second overhang section has a width larger than a width of said first overhang section.

8. The absorbent article defined by any one of Claims 1 through 7 wherein said second overhang section has a color different from that of said first overhang section.

9. The absorbent article defined by any one of Claims 1 through 8 wherein a second outer end of said second overhang section in its overhang direction lies at a level above a first outer end of said first overhang section in its overhang direction.

10. The absorbent article defined by any one of Claims 1 through 9 wherein said second outer end of said second overhang section in said overhang direction is formed with a void space.
